# EUROPEAN PATENT SPECIFICATION

(11) **EP 3 044 193 B1**
(45) Date of publication and mention of the grant of the patent: **05.06.2019**
(21) Application number: 14844001.9
(22) Date of filing: 03.09.2014
(51) Int. Cl.: C07C 2/78, C07C 5/09

(54) **PRODUCTION OF OLEFINS FROM A METHANE CONVERSION PROCESS**
HERSTELLUNG VON OLEFINEN AUS EINEM METHANUMWANDLUNGSVERFAHREN
PRODUCTION D'OLÉFINES À PARTIR D'UN PROCÉDÉ DE CONVERSION DE MÉTHANE

(30) Priority: 10.09.2013 US 201314022943
(43) Date of publication of application: 20.07.2016
(73) Proprietor: UOP LLC, Des Plaines, Illinois 60017-5017 (US)
(72) Inventor: NICHOLAS, Christopher P., Des Plaines, Illinois 60017-5017 (US)
(74) Representative: Boult Wade Tennant LLP
(86) International application number: PCT/US2014/053818
(87) International publication number: WO 2015/038377

(56) References cited:
- EP-B1- 0 909 216
- US-A- 2 958 716
- US-A- 4 724 272
- US-A- 5 177 282
- US-A1- 2005 065 391
- US-A1- 2005 288 471
- US-A1- 2006 173 224
- US-A1- 2010 249 474

## Description

### STATEMENT OF PRIORITY

This application claims priority to U.S. Application No. 14/022,943 which was filed September 10, 2013.

### FIELD OF THE INVENTION

A process is disclosed for the production of C6 to C12 olefins from the conversion of methane to acetylene using a supersonic flow reactor. More particularly, the process is for the production of olefins from methane.

### BACKGROUND OF THE INVENTION

The use of plastics and rubbers are widespread in today's world. The production of these plastics and rubbers are from the polymerization of monomers which are generally produced from petroleum. The monomers are generated by the breakdown of larger molecules to smaller molecules which can be modified. The monomers are then reacted to generate larger molecules comprising chains of the monomers. An important example of these monomers are light olefins, including ethylene and propylene, which represent a large portion of the worldwide demand in the petrochemical industry. Light olefins, and other monomers, are used in the production of numerous chemical products via polymerization, oligomerization, alkylation and other well-known chemical reactions. Producing large quantities of light olefin material in an economical manner, therefore, is a focus in the petrochemical industry. These monomers are essential building blocks for the modern petrochemical and chemical industries. The main source for these materials in present day refining is the steam cracking of petroleum feeds.

A principal means of production is the cracking of hydrocarbons brought about by heating a feedstock material in a furnace has long been used to produce useful products, including for example, olefin products. For example, ethylene, which is among the more important products in the chemical industry, can be produced by the pyrolysis of feedstocks ranging from light paraffins, such as ethane and propane, to heavier fractions such as naphtha. Typically, the lighter feedstocks produce higher ethylene yields (50-55% for ethane compared to 25-30% for naphtha); however, the cost of the feedstock is more likely to determine which is used. Historically, naphtha cracking has provided the largest source of ethylene, followed by ethane and propane pyrolysis, cracking, or dehydrogenation. Due to the large demand for ethylene and other light olefinic materials, however, the cost of these traditional feeds has steadily increased.

Energy consumption is another cost factor impacting the pyrolytic production of chemical products from various feedstocks. Over the past several decades, there have been significant improvements in the efficiency of the pyrolysis process that have reduced the costs of production. In a typical or conventional pyrolysis plant, a feedstock passes through a plurality of heat exchanger tubes where it is heated externally to a pyrolysis temperature by the combustion products of fuel oil or natural gas and air. One of the more important steps taken to minimize production costs has been the reduction of the residence time for a feedstock in the heat exchanger tubes of a pyrolysis furnace. Reduction of the residence time increases the yield of the desired product while reducing the production of heavier by-products that tend to foul the pyrolysis tube walls. However, there is little room left to improve the residence times or overall energy consumption in traditional pyrolysis processes.

More recent attempts to decrease light olefin production costs include utilizing alternative processes and/or feedstreams. In one approach, hydrocarbon oxygenates and more specifically methanol or dimethylether (DME) are used as an alternative feedstock for producing light olefin products. Oxygenates can be produced from available materials such as coal, natural gas, recycled plastics, various carbon waste streams from industry and various products and by-products from the agricultural industry. Making methanol and other oxygenates from these types of raw materials is well established and typically includes one or more generally known processes such as the manufacture of synthesis gas using a nickel or cobalt catalyst in a steam reforming step followed by a methanol synthesis step at relatively high pressure using a copper-based catalyst.

Once the oxygenates are formed, the process includes catalytically converting the oxygenates, such as methanol, into the desired light olefin products in an oxygenate to olefin (OTO) process. Techniques for converting oxygenates, such as methanol to light olefins (MTO), are described in United States Patent No. 4,387,263, which discloses a process that utilizes a catalytic conversion zone containing a zeolitic type catalyst. United States Patent No. 4,587,373 discloses using a zeolitic catalyst like ZSM-5 for purposes of making light olefins. United States Patent Nos. 5,095,163; 5,126,308 and 5,191,141 on the other hand, disclose an MTO conversion technology utilizing a non-zeolitic molecular sieve catalytic material, such as a metal aluminophosphate (ELAPO) molecular sieve. OTO and MTO processes, while useful, utilize an indirect process for forming a desired hydrocarbon product by first converting a feed to an oxygenate and subsequently converting the oxygenate to the hydrocarbon product. This indirect route of production is often associated with energy and cost penalties, often reducing the advantage gained by using a less expensive feed material. In addition, some oxygenates, such as vinyl acetate or acrylic acid, are also useful chemicals and can be used as polymer building blocks.

Recently, attempts have been made to use pyrolysis to convert natural gas to ethylene. US Patent No. 7,183,451 discloses heating natural gas to a temperature at which a fraction is converted to hydrogen and a hydrocarbon product such as acetylene or ethylene. The product stream is then quenched to stop further reaction and subsequently reacted in the presence of a catalyst to form liquids to be transported. The liquids ultimately produced include naphtha, gasoline, or diesel. While this method may be effective for converting a portion of natural gas to acetylene or ethylene, it is estimated that this approach will provide only a 40% yield of acetylene from a methane feed stream. While it has been identified that higher temperatures in conjunction with short residence times can increase the yield, technical limitations prevent further improvement to this process in this regard.

US 2005/0065391 describes a process for the conversion of natural gas to hydrocarbon liquids.

While the foregoing traditional pyrolysis systems provide solutions for converting ethane and propane into other useful hydrocarbon products, they have proven either ineffective or uneconomical for converting methane into these other products, such as, for example ethylene. While MTO technology is promising, these processes can be expensive due to the indirect approach of forming the desired product. Due to continued increases in the price of feeds for traditional processes, such as ethane and naphtha, and the abundant supply and corresponding low cost of natural gas and other methane sources available, for example the more recent accessibility of shale gas, it is desirable to provide commercially feasible and cost effective ways to use methane as a feed for producing ethylene and other useful hydrocarbons.

### SUMMARY OF THE INVENTION

In the first aspect there is provided a method for producing olefins as defined in claim 1.

A method for producing acetylene according to one aspect is provided. The method generally includes introducing a feed stream portion of a hydrocarbon stream including methane into a supersonic reactor. The method also includes pyrolyzing the methane in the supersonic reactor to form a reactor effluent stream portion of the hydrocarbon stream including acetylene. The method further includes treating at least a portion of the hydrocarbon stream in a process for producing higher value products. The method further includes passing the reactor effluent stream to a hydroprocessing reactor to form a second process stream comprising olefins.

According to another described aspect, a method for controlling a contaminant level in a hydrocarbon stream in the production of acetylene from a methane feed stream is provided. The method includes introducing a feed stream portion of a hydrocarbon stream including methane into a supersonic reactor. The method also includes pyrolyzing the methane in the supersonic reactor to form a reactor effluent stream portion of the hydrocarbon stream including acetylene. The method further includes maintaining the concentration of carbon monoxide in at least a portion of the process stream to below 1000 ppm by vol.

According to another described aspect, a system is provided for producing acetylene from a methane feed stream. The system includes a supersonic reactor for receiving a methane feed stream and configured to convert at least a portion of methane in the methane feed stream to acetylene through pyrolysis and to emit an effluent stream including the acetylene. The system also includes a hydrocarbon conversion zone in communication with the supersonic reactor and configured to receive the effluent stream and convert at least a portion of the acetylene therein to another hydrocarbon compound in a product stream. In one aspect, the process can include multiple hydrocarbon conversion zones, or a hydrocarbon conversion zone can include more than one type of hydrocarbon conversion process. The system includes a hydrocarbon stream line for transporting the methane feed stream, the reactor effluent stream, and the product stream. The system further includes a contaminant removal zone in communication with the hydrocarbon stream line for removing carbon oxides, and in particular carbon monoxide, from one of the methane feed stream, the effluent stream, and the product stream.

Other objects, advantages and applications of the present invention will become apparent to those skilled in the art from the following detailed description and drawings.

### BRIEF DESCRIPTION OF THE DRAWINGS

FIG. 1 is a side cross-sectional view of a supersonic reactor in accordance with various embodiments described herein;
FIG. 2 is a schematic view of a system for converting methane into acetylene and other hydrocarbon products in accordance with various embodiments described herein;
FIG. 3 is a schematic view of the general process for generating olefins from methane.

### DETAILED DESCRIPTION OF THE INVENTION

One proposed alternative to the previous methods of producing hydrocarbon products that has not gained much commercial traction includes passing a hydrocarbon feedstock into a supersonic reactor and accelerating it to supersonic speed to provide kinetic energy that can be transformed into heat to enable an endothermic pyrolysis reaction to occur. Variations of this process are set out in U.S. Pat. Nos. 4,136,015 and 4,724,272, and Russian Patent No. SU 392723A. These processes include combusting a feedstock or carrier fluid in an oxygen-rich environment to increase the temperature of the feed and accelerate the feed to supersonic speeds. A shock wave is created within the reactor to initiate pyrolysis or cracking of the feed. In particular, the hydrocarbon feed to the reactor comprises a methane feed. The methane feed is reacted to generate an intermediate process stream which is then further processed to generate a hydrocarbon product stream. A particular hydrocarbon product stream of interest is olefins, and in particular light olefins.

More recently, US Patent Nos. 5,219,530 and 5,300,216 have suggested a similar process that utilizes a shock wave reactor to provide kinetic energy for initiating pyrolysis of natural gas to produce acetylene. More particularly, this process includes passing steam through a heater section to become superheated and accelerated to a nearly supersonic speed. The heated fluid is conveyed to a nozzle which acts to expand the carrier fluid to a supersonic speed and lower temperature. An ethane feedstock is passed through a compressor and heater and injected by nozzles to mix with the supersonic carrier fluid to turbulently mix together at a Mach 2.8 speed and a temperature of 427°C. The temperature in the mixing section remains low enough to restrict premature pyrolysis. The shockwave reactor includes a pyrolysis section with a gradually increasing cross-sectional area where a standing shock wave is formed by back pressure in the reactor due to flow restriction at the outlet. The shock wave rapidly decreases the speed of the fluid, correspondingly rapidly increasing the temperature of the mixture by converting the kinetic energy into heat. This immediately initiates pyrolysis of the ethane feedstock to convert it to other products. A quench heat exchanger then receives the pyrolized mixture to quench the pyrolysis reaction.

Methods and systems for converting hydrocarbon components in methane feed streams using a supersonic reactor are generally disclosed. As used herein, the term "methane feed stream" includes any feed stream comprising methane. The methane feed streams provided for processing in the supersonic reactor generally include methane and form at least a portion of a process stream that includes at least one contaminant. The methods and systems presented herein remove or convert the contaminant in the process stream and convert at least a portion of the methane to a desired product hydrocarbon compound to produce a product stream having a reduced contaminant level and a higher concentration of the product hydrocarbon compound relative to the feed stream. By one approach, a hydrocarbon stream portion of the process stream includes the contaminant and methods and systems presented herein remove or convert the contaminant in the hydrocarbon stream.

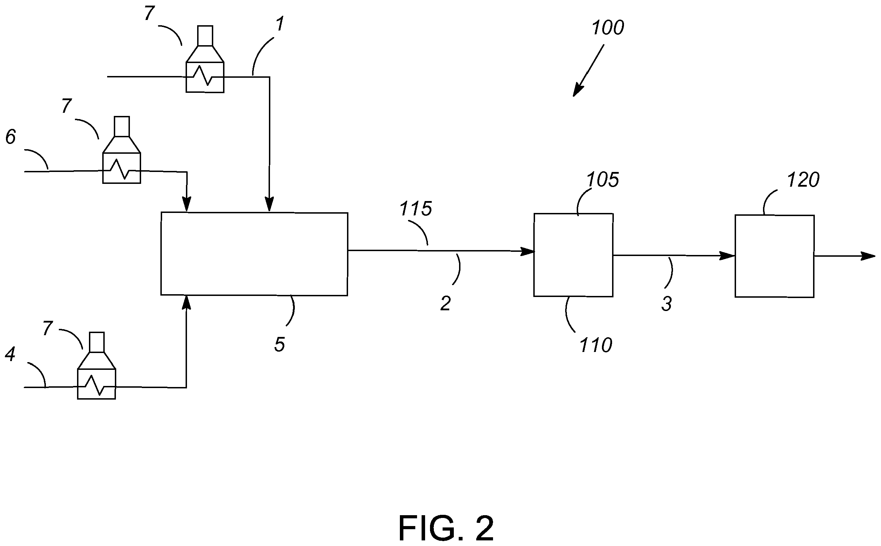

The term "hydrocarbon stream" as used herein refers to one or more streams that provide at least a portion of the methane feed stream entering the supersonic reactor as described herein or are produced from the supersonic reactor from the methane feed stream, regardless of whether further treatment or processing is conducted on such hydrocarbon stream. With reference to the example illustrated in FIG. 2, the "hydrocarbon stream" may include the methane feed stream 1, a supersonic reactor effluent stream 2, a desired product stream 3 exiting a downstream hydrocarbon conversion process or any intermediate or byproduct streams formed during the processes described herein. The hydrocarbon stream may be carried via a process stream line 115, as shown in FIG. 2, which includes lines for carrying each of the portions of the process stream described above. The term "process stream" as used herein includes the "hydrocarbon stream" as described above, as well as it may include a carrier fluid stream, a fuel stream 4, an oxygen source stream 6, or any streams used in the systems and the processes described herein. The process stream may be carried via a process stream line 115, which includes lines for carrying each of the portions of the process stream described above. As illustrated in FIG. 2, any of methane feed stream 1, fuel stream 4, and oxygen source stream 6, may be preheated, for example, by one or more heaters 7.

The term "communication" means fluid communication where material is permitted to flow between enumerated components. The term "downstream communication" means that at least a portion of the material flowing to the subject in downstream communication may operatively flow from the object with which it communicates. The term "upstream communication" means that at least a portion of the material flowing from the subject in upstream communication may operatively flow to the object with which it communicates.

Prior attempts to convert light paraffin or alkane feed streams, including ethane and propane feed streams, to other hydrocarbons using supersonic flow reactors have shown promise in providing higher yields of desired products from a particular feed stream than other more traditional pyrolysis systems. Specifically, the ability of these types of processes to provide very high reaction temperatures with very short associated residence times offers significant improvement over traditional pyrolysis processes. It has more recently been realized that these processes may also be able to convert methane to acetylene and other useful hydrocarbons, whereas more traditional pyrolysis processes were incapable or inefficient for such conversions.

The majority of previous work with supersonic reactor systems, however, has been theoretical or research based, and thus has not addressed problems associated with practicing the process on a commercial scale. In addition, many of these prior disclosures do not contemplate using supersonic reactors to effectuate pyrolysis of a methane feed stream, and tend to focus primarily on the pyrolysis of ethane and propane. One problem that has recently been identified with adopting the use of a supersonic flow reactor for light alkane pyrolysis, and more specifically the pyrolysis of methane feeds to form acetylene and other useful products therefrom, includes negative effects that particular contaminants in commercial feed streams can create on these processes and/or the products produced therefrom. Previous work has not considered the need for product purity, especially in light of potential downstream processing of the reactor effluent stream. Product purity can include the separation of several products into separate process streams, and can also include treatments for removal of contaminants that can affect a downstream reaction, and downstream equipment.

In accordance with various embodiments disclosed herein, therefore, processes and systems for converting the methane to a product stream are presented. The methane is converted to an intermediate process stream comprising acetylene. The intermediate process stream is converted to a second process stream comprising either a hydrocarbon product, or a second intermediate hydrocarbon compound. The processing of the intermediate acetylene stream can include purification or separation of acetylene from by-products.

The removal of particular contaminants and/or the conversion of contaminants into less deleterious compounds has been identified to improve the overall process for the pyrolysis of light alkane feeds, including methane feeds, to acetylene and other useful products. In some instances, removing these compounds from the hydrocarbon or process stream has been identified to improve the performance and functioning of the supersonic flow reactor and other equipment and processes within the system. Removing these contaminants from hydrocarbon or process streams has also been found to reduce poisoning of downstream catalysts and adsorbents used in the process to convert acetylene produced by the supersonic reactor into other useful hydrocarbons, for example hydrogenation catalysts that may be used to convert acetylene into ethylene. Still further, removing certain contaminants from a hydrocarbon or process stream as set forth herein may facilitate meeting product specifications.

In accordance with one approach, the processes and systems disclosed herein are used to treat a hydrocarbon process stream, to remove a contaminant therefrom and convert at least a portion of methane to acetylene. The hydrocarbon process stream described herein includes the methane feed stream provided to the system, which includes methane and may also include ethane or propane. The methane feed stream may also include combinations of methane, ethane, and propane at various concentrations and may also include other hydrocarbon compounds. In one approach, the hydrocarbon feed stream includes natural gas. The natural gas may be provided from a variety of sources including, but not limited to, gas fields, oil fields, coal fields, fracking of shale fields, biomass, and landfill gas. In another approach, the methane feed stream can include a stream from another portion of a refinery or processing plant. For example, light alkanes, including methane, are often separated during processing of crude oil into various products and a methane feed stream may be provided from one of these sources. These streams may be provided from the same refinery or different refinery or from a refinery off gas. The methane feed stream may include a stream from combinations of different sources as well.

In accordance with the processes and systems described herein, a methane feed stream may be provided from a remote location or at the location or locations of the systems and methods described herein. For example, while the methane feed stream source may be located at the same refinery or processing plant where the processes and systems are carried out, such as from production from another on-site hydrocarbon conversion process or a local natural gas field, the methane feed stream may be provided from a remote source via pipelines or other transportation methods. For example a feed stream may be provided from a remote hydrocarbon processing plant or refinery or a remote natural gas field, and provided as a feed to the systems and processes described herein. Initial processing of a methane stream may occur at the remote source to remove certain contaminants from the methane feed stream. Where such initial processing occurs, it may be considered part of the systems and processes described herein, or it may occur upstream of the systems and processes described herein. Thus, the methane feed stream provided for the systems and processes described herein may have varying levels of contaminants depending on whether initial processing occurs upstream thereof.

In one example, the methane feed stream has a methane content ranging from 65 mol-% to 100 mol-% of the hydrocarbon feed. In another example, the concentration of methane in the hydrocarbon feed ranges from 80 mol-% to 100 mol-% of the hydrocarbon feed. In yet another example, the concentration of methane ranges from 90 mol-% to 100 mol-% of the hydrocarbon feed.

In one example, the concentration of ethane in the methane feed ranges from 0 mol-% to 35 mol-% and in another example from 0 mol-% to 10 mol-%. In one example, the concentration of propane in the methane feed ranges from 0 mol-% to 5 mol-% and in another example from 0 mol-% to 1 mol-%.

The methane feed stream may also include heavy hydrocarbons, such as aromatics, paraffinic, olefinic, and naphthenic hydrocarbons. These heavy hydrocarbons if present will likely be present at concentrations of between 0 mol-% and 100 mol-%. In another example, they may be present at concentrations of between 0 mol-% and 10 mol-% and may be present at between 0 mol-% and 2 mol-%.

An aspect of this invention is the production of olefins from the acetylene generated by the supersonic reactor. The reactor converts a methane stream through pyrolysis to generate a reactor effluent stream comprising acetylene. In one embodiment, the reactor effluent stream is passed to a hydrocarbon conversion zone. The hydrocarbon conversion zone is in downstream communication with the supersonic pyrolysis reactor effluent stream. In one embodiment, the hydrocarbon conversion zone is a hydroprocessing zone and the reactor effluent stream is passed to a hydroprocessing reactor to form a second process stream comprising olefins. In one embodiment, the reactor effluent stream is passed to a reactor effluent treating unit to remove carbon oxides in the reactor effluent stream. Carbon oxides include carbon monoxide (CO) and carbon dioxide (CO₂). In an alternate configuration, the reactor effluent stream is first passed through the hydrocarbon conversion zone, and then passed through the effluent treating unit to remove residual carbon oxides. The reactor effluent treating unit can remove other contaminants, especially oxidizing compounds such as CO to a level below at least 1 vol-%, and preferable to below a level of 100 vol-ppm. The removal of CO before further processing is to limit or prevent other reactions that can lead to a reduction in the yields of olefins. The treating unit can comprise an adsorber. Adsorbers are known to those skilled in the art, and can be designed to adsorb polar compounds from a mixture comprising polar and non-polar compounds. In one embodiment, the hydroprocessing reactor is a hydrogenation reactor for converting acetylene to ethylene in the presence of hydrogen. The hydroprocessing reactor effluent stream can be passed to a light olefins recovery unit to separate ethylene and other olefins from the hydroprocessing reactor effluent stream. In one embodiment, the hydrocarbon conversion zone is comprised of more than one type of hydrocarbon conversion. The reactor effluent treating unit may be within the hydrocarbon conversion zone or before the hydrocarbon conversion zone. In a preferred embodiment, the hydrocarbon conversion zone is comprised of a hydroprocessing zone and a hydrocarbon conversion zone, preferably an olefin conversion zone. Examples of olefin conversion zones include olefin dimerization, olefin oligomerization, olefin polymerization, olefin cracking, olefin metathesis and combinations thereof.

Pyrolysis reactor effluent can be converted in a hydrocarbon conversion zone to higher hydrocarbons with ZSM-5 or metal modified SAPO catalysts. ZSM-5 catalyst of the MFI zeotype has a three dimensional 10-membered ring pore structure and forms higher hydrocarbons including aromatics from the acetylene. The gross elemental composition, i.e. the carbon, hydrogen and oxygen (CHO) composition, from a shock wave pyrolysis reactor effluent gives a similar gross elemental composition from a methanol to olefin (MTO) conversion process. Given this composition, without being confined to any particular theory, it is believed that acetylene would react over the MFI based catalyst to form the active hydrocarbon pool on the catalyst with a high amount of hydrogen present, and thus generate light olefins and gasoline or aromatics from passing the acetylene stream to a reactor with an MFI catalyst. This presents a benefit of little or no clean-up downstream since there would be little or no residual oxygenates in the effluent stream, and can provide an attractive alternative to conventional methods of methane to olefins.

Control to produce a more selective group of hydrocarbons is desired for savings in processing and energy. The use of a shape selective catalyst can facilitate the production of olefins. A metal modified SAPO catalyst provides for shape selectivity, such that the amount of aromatics, or ring compounds, formed will be limited. A particular SAPO catalyst is SAPO-34 which should favor the production of light olefins from an acetylene feed stream. In one embodiment, the process includes a metal modified SAPO catalyst to generate light olefins. The metal, or metal oxide, uses one or more metals selected from IUPAC Groups 8, 9, 10, 11, 12, and 13. A preferred metal is one or more metals selected from gallium (Ga), platinum (Pt), and palladium (Pd), and the preferred SAPO is SAPO-34. Metal oxides that can be used include MoO3 and Mn2O3.

Conditions used in the hydrocarbon conversion zone with the ZSM-5 or metal modified SAPO catalyst include carrying out the process at elevated temperatures in order to form light olefins at a fast enough rate. Thus, the process should be carried out at a temperature of 300°C to 600°C, and preferably from 400°C to 550°C. The process may be carried out over a wide range of pressures including autogenous pressure. Thus, the pressure can vary from 100 kPa (absolute) to 8 MPa (absolute), with a preferred range from 100 kPa (absolute) to 1850 kPa (absolute) and preferably from 130 kPa (absolute) to 450 kPa (absolute). The operating conditions also include a weight hourly space velocity between 1 hr⁻¹ and 10 hr⁻¹.

Optionally, the supersonic pyrolysis reactor effluent may be diluted with an inert diluent in order to more efficiently convert the reactor effluent to olefins in the hydrocarbon conversion zone. Examples of the diluents which may be used are helium, argon, nitrogen, carbon monoxide, carbon dioxide, hydrogen, steam, methane and mixtures thereof. The amount of diluent used can vary considerably and is usually from 5 to 90 mole percent of the feedstock and preferably from 25 to 75 mole percent.

Hydrogenation reactors comprise a hydrogenation catalyst, and are operated at hydrogenation conditions to hydrogenate unsaturated hydrocarbons. Hydrogenation catalysts typically comprise a hydrogenation metal on a support, wherein the hydrogenation metal is preferably selected from a Group VIII metal in an amount between 0.01 and 2 wt. % of the catalyst. Preferably the metal is platinum (Pt), palladium (Pd), or a mixture thereof. The support is preferably a molecular sieve, and can include zeolites such as zeolite beta, MCM-22, MCM-36, mordenite, faujasites such as X-zeolites and Y-zeolites, including B-Y-zeolites and USY-zeolites; non-zeolitic solids such as silica-alumina, sulfated oxides such as sulfated oxides of zirconium, titanium, or tin, mixed oxides of zirconium, molybdenum, tungsten, phosphorus and chlorinated aluminium oxides or clays. Preferred supports are zeolites, including mordenite, zeolite beta, faujasites such as X-zeolites and Y-zeolites, including BY-zeolites and USY-zeolites. Mixtures of solid supports can also be employed.

In one embodiment, the present invention generates larger olefins. The hydroprocessing effluent stream is passed to a second reactor in the hydrocarbon conversion zone. In a preferred embodiment, the second reactor comprises an olefin conversion zone. The second reactor can include a dimerization reactor for generating butenes, an oligomerization reactor for generating larger olefins, an olefin metathesis reactor, an olefin polymerization reactor, an olefin cracking reactor, and other reactors or combinations thereof. The oligomerization reactor and process is operated to generate olefins in the C6 to C12 range, and in one embodiment, the process is operated to generate C6 and C8 olefins, and preferably 1-hexene and 1-octene, or a mixture of 1-hexene and 1-octene. The oligomerization unit is operated to generate 1-hexene and 1-octene in amounts greater than 10% yield based upon the ethylene content of the feedstream to the oligomerization unit. The oligomerization catalysts to produce 1-hexene and 1-octene can comprise any oligomerization catalyst. A particular oligomerization catalyst preferably has a silica base with a metal from Group VIIIB in the periodic table using Chemical Abstracts Service notations. In an aspect, the silica base may include alumina. The base is preferably amorphous. In an aspect, the catalyst exhibits low acidity, having a silicon-to-aluminum ratio of no less than 20 and preferably no less than 50. Typically, the silicon and aluminum will only be in the base, so the silicon-to-aluminum ratio will be the same for the catalyst as for the base. The metals can either be impregnated onto or ion exchanged with the silica-alumina base. Co-mulling is also contemplated. Nickel is the preferred metal and in an aspect nickel (II) is preferred. Metal contents range from 0.5wt% to 10wt% with a preferred range of 1wt% to 8wt%. Additionally, a suitable catalyst will have a surface area of between 50 and 500 m²/g as determined by nitrogen BET. Preferred oligomerization catalysts comprise organometallic catalysts. The organometallic catalysts preferably comprise a metal bonded to more than 1 organic ligand. Examples of preferred organometallic catalysts include Cr-PNP systems such as bis(diphenylphosphino)ethylamine Cr (III) chloride, Cr-SNS systems such as bis(dithioether)amine Cr (III) chloride, Cr-PNN systems such as diphenylphosphinotrimethylethylenediamine Cr (III) chloride, (Me3P)Cr[µ-(tBu)NPPh2]3Cr, chromium triazacyclohexane complexes, and Ti cyclopentadienyl based systems such as 1,1'-dimethylbenzylcyclopentadienyltitanium trichloride. Conditions to perform the olefin oligomerization process to generate C6 and C8 olefins include a temperature from 25°C to 200°C with a preferred range from 50°C to 150°C. The pressure used ranges from 100 kPa (absolute) to 2 MPa (absolute). PNP complexes are organic complexes that allow ligand formation with R₂P-Cₓ-NH-Cₓ-PR₂ structure with x typically equal to 2 or 3, although these carbon atoms may be part of a larger group such as an aryl group, and R is a hydrocarbon group although the R groups may be the same or different. Likewise, SNS complexes are organic complexes that allow ligand formation with RS-Cₓ-NH-Cₓ-SR structure, and PNN complexes are organic complexes that allow ligand formation with R₂P-Cₓ-NH-Cₓ-NR₂ structure.

In one embodiment, the olefin oligomerization process generates C6-C12 olefins in the gasoline boiling point range. The oligomerization of light olefins is performed for the production of a high quality gasoline product, comprising highly branched alkanes and without aromatic compounds, or with very low amounts of aromatic compounds, or aromatics content of less than 1 wt. %. In an embodiment, the present invention provides a process for the oligomerization of light olefins derived from pyrolysis reactor effluent by conversion over a catalyst comprising a zeolite and a binder, wherein the zeolite has a structure selected from the group consisting of MFI, MEL, ITH, IMF, TUN, FER, BEA, FAU, BPH, MEI, MSE, MWW, UZM-8, MOR, OFF, MTW, TON, MTT, AFO, ATO, and AEL, and mixtures thereof, wherein the catalyst has been treated with a phosphorous containing reagent selected from the group consisting of phosphate compounds, phosphite compounds, phosphorus oxytrichloride, and mixtures thereof, thereby forming a treated catalyst having a micropore volume less than 50%, and a crystallinity greater than 50% of the untreated catalyst as measured by standardized BET adsorption theory and x-ray diffraction techniques, respectively. The catalyst, before treatment, will preferably have an initial micropore volume of at least 0.05 ml N₂/g.

Here, the process conditions to be used include a temperature of between 70 and 300°C or preferably between 90°C and 220°C, pressure greater than 2MPa and preferably greater than 3MPa up to 11MPa. Preferably, the zeolite is of the MTW or MTT zeotypes. Zeotype codes are generated by the International Zeolite Association Structure Commission as maintained in the IZA Database (IZA Database of Zeolite Structures, available at www.iza-structure.org). Preferably the binder is an alumina (Al₂O₃) binder. During the treatment with the phosphorous containing reagent, preferably at least a portion of the Al₂O₃ binder is converted to a crystalline aluminum phosphate (AlPO₄).

In one embodiment, the present invention is directed to generating propylene. A first portion of the hydroprocessing effluent stream, comprising ethylene, is passed to a dimerization reactor to generate a dimerization effluent comprising butene. The dimerization effluent and a second portion of the hydroprocessing effluent stream is passed to a metathesis reactor for converting the ethylene and butenes to generate a metathesis effluent stream comprising propylene. The metathesis effluent stream can be passed to the light olefins recovery unit to generate an ethylene product stream, a propylene product stream and a heavies stream for recycle. Catalysts which are active for the metathesis of olefins and which can be used in the process of this invention are of a generally known type. In this regard, reference is made to JOURNAL OF CATALYSIS, 13 (1969) pages 99-114, to APPLIED CATALYSIS, 10 (1984) pages 29-229 and to CATALYSIS REVIEW, 3 (1) (1970) pages 37-60. However, a preferred catalyst comprises tungsten disposed on a support comprising silica having a surface area from 400 m²/g to 550 m²/g and an average pore diameter from 45Å to 170Å. The tungsten is present in an amount from 1 to 10wt% and preferably from 4 to 7 wt%. Regardless of the source, the silica will have a surface area, either as received or after an optional acid washing step in the catalyst preparation procedure, of at least 250 square meters per gram (m²/g), with particularly advantageous results being obtained in the range from 250 m²/g to 600 m²/g, and often from 400 m²/g to 550 m²/g. The average pore diameter of the silica, either as received or after acid washing, is preferably at least 40 angstroms (Å), with exemplary average pore diameters being in the range from 45 Å to 170 Å, and even in the range from 45 Å to 100 Å. Surface area and average pore diameter are measured according to the Brunauer, Emmett and Teller (BET) method based on nitrogen adsorption (ASTM D1993-03(2008)). As discussed above, it is thought that the combination of surface area and average pore diameter characteristics act in synergy, in the catalysts for olefin metathesis as described herein, to provide highly effective kinetic and size selective properties that simultaneously benefit both conversion and product selectivity. Acid washing of silica supports, used for supported tungsten oxide catalysts, significantly improves the activity of the resulting catalyst (i.e., its conversion level at a given temperature) for the metathesis of olefins, without significantly compromising its selectivity to the desired conversion product(s). Acid washing of the silica support involves contacting the silica used in the support with an acid, including an organic acid or an inorganic acid. Particular inorganic acids include nitric acid, sulfuric acid, and hydrochloric acid, with nitric acid and hydrochloric acid being preferred. The acid concentration in aqueous solution, used for the acid washing, is generally in the range from 0.05 molar (M) to 3 M, and often from 0.1 M to 1 M. The acid washing can be performed under static conditions (e.g., batch) or flowing conditions (e.g., once-through, recycle, or with a combined flow of make-up and recycle solution).

Representative contacting conditions used to provide an acid washed silica support include a temperature generally from 20°C to 120°C, typically from 30°C to 100°C, and often from 50°C to 90°C. The contacting time, or a duration over which the silica support is contacted with the acid at a temperature within any of these ranges, is generally from 10 minutes to 5 hours, and often from 30 minutes to 3 hours. Normally, it is preferred that the conditions of contacting used to perform acid washing of the silica support result in one or more observed physical or compositional changes in the support that can be verified analytically. For example, it has been determined that effective acid washing is generally accompanied by an increase in the surface area (BET method as indicated above) of the silica support of at least 5% (e.g., from 5% to 20%), and often at least 10% (e.g., from 10% to 15%), relative to the surface area of the support prior to being acid washed. Another change is a decrease in the amount of aluminum (as aluminum metal) of the acid washed support, relative to the amount in the support prior to being acid washed. The reduction in aluminum can be verified using inductively coupled plasma (ICP) techniques, known in the art, for the determination of trace metals. Generally, the amount of aluminum is decreased by at least 35% (e.g., from 35% to 90%) and often at least 50% (e.g., from 50% to 80%). A third change is a decrease in the average pore diameter of the acid washed silica support, relative to the average pore diameter of the support prior to being acid washed. In general, the pore volume is decreased by at least 5%, and often by at least 10%. The feedstock may be contacted with the catalyst at a temperature from 300°C to 600°C, a pressure from 1 MPa (absolute) to 8 MPa (absolute), and a weight hourly space velocity from 1 hr⁻¹ to 10 hr⁻¹.

In an embodiment, the present invention is directed to generating propylene. In this embodiment, the second reactor in the hydrocarbon conversion zone comprises an olefin metathesis reactor and embodiments of the invention relate to olefin metathesis processes comprising contacting a hydrocarbon feedstock with a catalyst comprising a solid support and a tungsten hydride bonded to alumina present in the support. The hydroprocessing reactor effluent comprising ethylene is in upstream communication with the catalyst comprising tungsten hydride on alumina. Moreover, the catalyst comprising tungsten hydride bonded to alumina is in downstream communication with the supersonic methane pyrolysis reactor effluent. The olefin metathesis reactor operates at temperatures from 100°C to 250°C with temperatures of between 120°C and 200°C preferred. Pressures of from 1 atmosphere to 10 atmospheres can be utilized. The ethylene is converted over the tungsten hydride bonded to alumina catalyst to propylene with a selectivity greater than 90wt%, preferably greater than 97wt% and most preferably greater than 99wt%. In an embodiment, a first portion of the hydroprocessing reactor effluent is passed to the olefin metathesis reactor and a second portion of the hydroprocessing reactor effluent is passed to a dimerization reactor. In this embodiment, the dimerization reactor effluent is then passed to the olefin metathesis reactor. The olefin metathesis reactor is in downstream communication with the hydroprocessing reactor effluent and the dimerization reactor effluent. In an embodiment, the ratio of the ethylene from the hydroprocessing reactor effluent to the butene from the dimerization reactor effluent is from 0.2:1 to less than 1:1 and preferably is from 0.2:1 to 0.8:1 or most preferably from 0.2:1 to 0.5:1. According to any of the above embodiments, the catalyst may comprise tungsten in an amount from 1% to 10% by weight and the support may have a surface area surface area from 100 m²/g to 450 m²/g.

In another embodiment, the present invention is directed to generating larger olefins having 5 or more carbon atoms. The hydroprocessing stream comprising ethylene is passed to an oligomerization reactor, where the reactor has an oligomerization catalyst and is operated at oligomerization conditions to generate an oligomerization effluent stream comprising olefins having 5 or more carbon atoms. The oligomerization effluent stream is passed to an olefins recovery unit for separation of the different olefins. The olefins recovery unit can comprise multiple fractionation units, multiple adsorption separation units, or some combination thereof for separating and recovering desired olefins in olefin product streams, and recycling undesired olefins in a recycle stream.

In one embodiment, the process can include passing the oligomerization effluent stream to a light olefins recovery unit to generate a light olefins product stream and a heavies stream comprising C4+ hydrocarbons. The heavies stream is passed to an olefin cracking unit to generate light olefins, and the olefin cracking unit effluent is passed to the light olefins recovery unit. This embodiment can also pass recycle streams, comprising heavier olefins, from other processes to the olefin cracking unit.

One aspect of the invention is a system for the generation of olefins. The invention includes a supersonic reactor having an inlet for receiving a methane feed stream and configured to convert at least a portion of methane in the methane feed stream to acetylene through pyrolysis and to emit an effluent stream including the acetylene. The invention includes an acetylene enrichment unit having an inlet in fluid communication with the reactor outlet, and an outlet for an acetylene enriched effluent. The invention includes a hydrocarbon conversion zone having an inlet in communication with the acetylene enrichment unit outlet and configured to receive the effluent stream and convert at least a portion of the acetylene therein to a process stream comprising olefins, and having an outlet for the process stream, and an olefin recovery unit having an inlet in fluid communication with the hydrocarbon conversion zone outlet. Thus, the hydrocarbon conversion zone comprising one or more hydrocarbon conversion reactors is in downstream communication with the supersonic methane pyrolysis reactor. One aspect of the system can further include a contaminant removal zone having an inlet in fluid communication with the acetylene enrichment zone outlet, and an outlet in fluid communication with the hydrocarbon conversion zone inlet, for removing contaminants that can adversely affect downstream catalysts and processes. One contaminant to be removed is CO to a level of less than 0.1 mole%, and preferably to a level of less than 100 ppm by vol. A particular aspect is where the hydrocarbon conversion zone comprises a hydrogenation reactor for converting acetylene to ethylene. An additional aspect of the invention is where the system can include a second contaminant removal zone having an inlet in fluid communication with the methane feed stream and an outlet in fluid communication with the supersonic reactor inlet.

In one embodiment, the present invention comprises a process for generating polyacetylenes. The process includes passing a methane feedstream to a supersonic reactor where the methane is pyrolyzed to generate a process stream comprising acetylene. The process stream is passed to a polymerization reactor to form a process stream comprising polyacetylene. The process can include an acetylene enrichment unit to remove contaminants such as carbon monoxide, and a polyacetylene recovery unit to separate polyacetylenes from the polyacetylene process stream.

The process for forming acetylene from the methane feed stream described herein utilizes a supersonic flow reactor for pyrolyzing methane in the feed stream to form acetylene. The supersonic flow reactor may include one or more reactors capable of creating a supersonic flow of a carrier fluid and the methane feed stream and expanding the carrier fluid to initiate the pyrolysis reaction. In one approach, the process may include a supersonic reactor as generally described in U.S. Patent No. 4,724,272, which is incorporated herein by reference, in their entirety. In another approach, the process and system may include a supersonic reactor such as described as a "shock wave" reactor in U.S. Patent Nos. 5,219,530 and 5,300,216, which are incorporated herein by reference, in their entirety. In yet another approach, the supersonic reactor described as a "shock wave" reactor may include a reactor such as described in "Supersonic Injection and Mixing in the Shock Wave Reactor" Robert G. Cerff, University of Washington Graduate School, 2010.

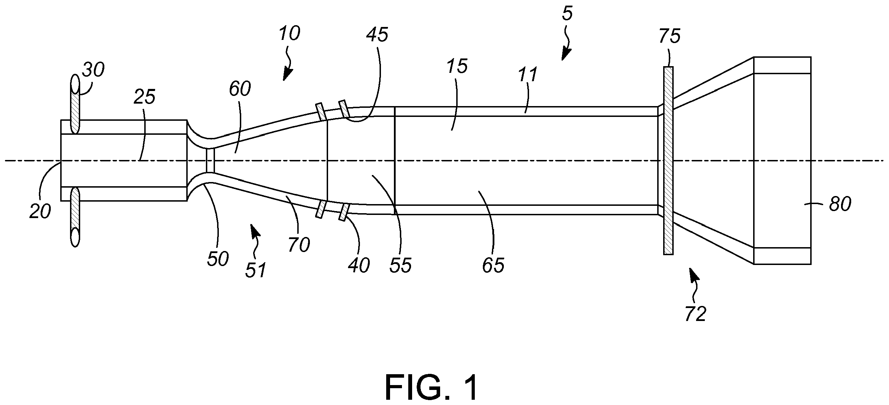

While a variety of supersonic reactors may be used in the present process, an exemplary reactor 5 is illustrated in FIG. 1. Referring to FIG. 1, the supersonic reactor 5 includes a reactor vessel 10 generally defining a reactor chamber 15. While the reactor 5 is illustrated as a single reactor, it should be understood that it may be formed modularly or as separate vessels. A combustion zone or chamber 25 is provided for combusting a fuel to produce a carrier fluid with the desired temperature and flowrate. The reactor 5 may optionally include a carrier fluid inlet 20 for introducing a supplemental carrier fluid into the reactor. One or more fuel injectors 30 are provided for injecting a combustible fuel, for example hydrogen, into the combustion chamber 25. The same or other injectors may be provided for injecting an oxygen source into the combustion chamber 25 to facilitate combustion of the fuel. The fuel and oxygen are combusted to produce a hot carrier fluid stream typically having a temperature of from 1200°C to 3500°C in one example, between 2000°C and 3500°C in another example, and between 2500°C and 3200°C in yet another example. According to one example the carrier fluid stream has a pressure of 100 kPa or higher, greater than 200 kPa in another example, and greater than 400 kPa in another example.

The hot carrier fluid stream from the combustion zone 25 is passed through a converging-diverging nozzle 50 to accelerate the flowrate of the carrier fluid to above mach 1.0 in one example, between mach 1.0 and mach 4.0 in another example, and between mach 1.5 and 3.5 in another example. In this regard, the residence time of the fluid in the reactor portion of the supersonic flow reactor is between 0.5 to 100 ms (milliseconds) in one example, 1 to 50 ms in another example, and 1.5 to 20 ms in another example.

A feedstock inlet 40 is provided for injecting the methane feed stream into the reactor 5 to mix with the carrier fluid. The feedstock inlet 40 may include one or more injectors 45 for injecting the feedstock into the nozzle 50, a mixing zone 55, an expansion zone 60, or a reaction zone or chamber 65. The injector 45 may include a manifold, including for example a plurality of injection ports.

In one approach, the reactor 5 may include a mixing zone 55 for mixing of the carrier fluid and the feed stream. In another approach, no mixing zone is provided, and mixing may occur in the nozzle 50, expansion zone 60, or reaction zone 65 of the reactor 5. An expansion zone 60 includes a diverging wall 70 to produce a rapid reduction in the velocity of the gases flowing therethrough, to convert the kinetic energy of the flowing fluid to thermal energy to further heat the stream to cause pyrolysis of the methane in the feed, which may occur in the expansion section 60 and/or a downstream reaction section 65 of the reactor. The fluid is quickly quenched in a quench zone 72 to stop the pyrolysis reaction from further conversion of the desired acetylene product to other compounds. Spray bars 75 may be used to introduce a quenching fluid, for example water or steam into the quench zone 72.

The reactor effluent exits the reactor via outlet 80 and as mentioned above forms a portion of the hydrocarbon stream. The effluent will include a larger concentration of acetylene than the feed stream and a reduced concentration of methane relative to the feed stream. The reactor effluent stream may also be referred to herein as an acetylene stream as it includes an increased concentration of acetylene. The acetylene may be an intermediate stream in a process to form another hydrocarbon product or it may be further processed and captured as an acetylene product stream. In one example, the reactor effluent stream has an acetylene concentration prior to the addition of quenching fluids ranging from 2 mol-% to 30 mol-%. In another example, the concentration of acetylene ranges from 5 mol-% to 25 mol-% and from 8 mol-% to 23 mol-% in another example.

In one example, the reactor effluent stream has a reduced methane content relative to the methane feed stream ranging from 15 mol-% to 95 mol-%. In another example, the concentration of methane ranges from 40 mol-% to 90 mol-% and from 45 mol-% to 85 mol-% in another example.

In one example the yield of acetylene produced from methane in the feed in the supersonic reactor is between 40 % and 95 %. In another example, the yield of acetylene produced from methane in the feed stream is between 50 % and 90 %. Advantageously, this provides a better yield than the estimated 40% yield achieved from previous, more traditional, pyrolysis approaches.

By one approach, the reactor effluent stream is reacted to form another hydrocarbon compound. In this regard, the reactor effluent portion of the hydrocarbon stream may be passed from the reactor outlet to a downstream hydrocarbon conversion process for further processing of the stream. While it should be understood that the reactor effluent stream may undergo several intermediate process steps, such as, for example, water removal, adsorption, and/or absorption to provide a concentrated acetylene stream, these intermediate steps will not be described in detail herein.

Referring to FIG. 2, the reactor effluent stream having a higher concentration of acetylene may be passed to a downstream hydrocarbon conversion zone 100 where the acetylene may be converted to form another hydrocarbon product. The hydrocarbon conversion zone 100 may include a hydrocarbon conversion reactor 105 for converting the acetylene to another hydrocarbon product. While FIG. 2 illustrates a process flow diagram for converting at least a portion of the acetylene in the effluent stream to ethylene through hydrogenation in hydrogenation reactor 110, it should be understood that the hydrocarbon conversion zone 100 may include a variety of other hydrocarbon conversion processes instead of or in addition to a hydrogenation reactor 110, or a combination of hydrocarbon conversion processes. Similarly, it illustrated in FIG. 2 may be modified or removed and are shown for illustrative purposes and not intended to be limiting of the processes and systems described herein. Specifically, it has been identified that several other hydrocarbon conversion processes, other than those disclosed in previous approaches, may be positioned downstream of the supersonic reactor 5, including processes to convert the acetylene into other hydrocarbons, including, but not limited to: alkenes, alkanes, methane, acrolein, acrylic acid, acrylates, acrylamide, aldehydes, polyacetylides, benzene, toluene, styrene, xylenes, aniline, cyclohexanone, caprolactam, propylene, butadiene, butyne diol, butandiol, C2-C4 hydrocarbon compounds, ethylene glycol, diesel fuel, diacids, diols, pyrrolidines, and pyrrolidones.

A contaminant removal zone 120 for removing one or more contaminants from the hydrocarbon or process stream may be located at various positions along the hydrocarbon or process stream depending on the impact of the particular contaminant on the product or process and the reason for the contaminants removal, as described further below. For example, particular contaminants have been identified to interfere with the operation of the supersonic flow reactor 5 and/or to foul components in the supersonic flow reactor 5. Thus, according to one approach, a contaminant removal zone is positioned upstream of the supersonic flow reactor in order to remove these contaminants from the methane feed stream prior to introducing the stream into the supersonic reactor. Other contaminants have been identified to interfere with a downstream processing step or hydrocarbon conversion process, in which case the contaminant removal zone may be positioned upstream of the supersonic reactor or between the supersonic reactor and the particular downstream processing step at issue. Still other contaminants have been identified that should be removed to meet particular product specifications. Where it is desired to remove multiple contaminants from the hydrocarbon or process stream, various contaminant removal zones may be positioned at different locations along the hydrocarbon or process stream. In still other approaches, a contaminant removal zone may overlap or be integrated with another process within the system, in which case the contaminant may be removed during another portion of the process, including, but not limited to the supersonic reactor 5 or the downstream hydrocarbon conversion zone 100. This may be accomplished with or without modification to these particular zones, reactors or processes. While the contaminant removal zone 120 illustrated in FIG. 2 is shown positioned downstream of the hydrocarbon conversion reactor 105, it should be understood that the contaminant removal zone 120 in accordance herewith may be positioned upstream of the supersonic flow reactor 5, between the supersonic flow reactor 5 and the hydrocarbon conversion zone 100, or downstream of the hydrocarbon conversion zone 100 as illustrated in FIG. 2 or along other streams within the process stream, such as, for example, a carrier fluid stream, a fuel stream, an oxygen source stream, or any streams used in the systems and the processes described herein.

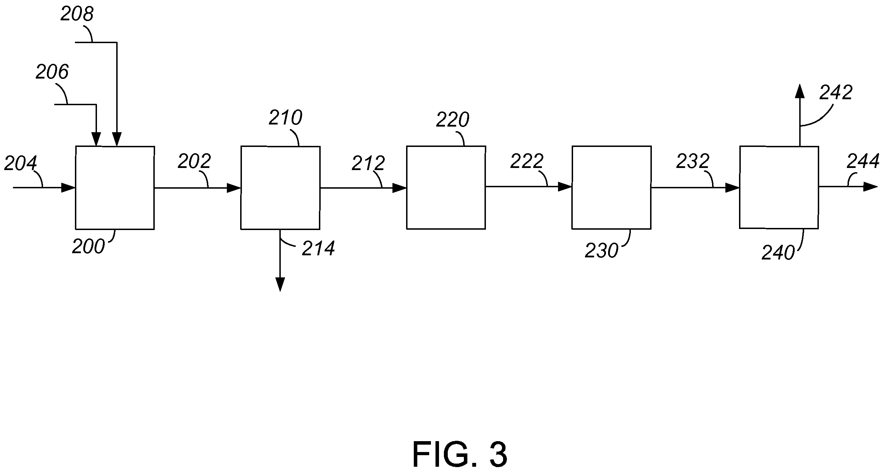

Referring to FIG. 3, the process includes passing a hydrocarbon feedstream 204 comprising methane to a supersonic reactor 200. The supersonic reactor 200 includes a fuel 206 and an oxidizing stream 208 for generating the supersonic flow through a combustion process. The fuel can be hydrogen or other suitable fuel, and the oxidizing stream will typically be an oxygen rich stream, such as oxygen, air or oxygen enriched air. The reactor 200 generates an effluent stream 202 comprising acetylene, and other products of combustion. Depending on the process chosen for converting the acetylene to other hydrocarbons, the removal of carbon oxides is performed for protecting downstream processes. The effluent stream 202 is passed to a carbon oxide removal zone 210 for removing CO and CO2, to generate an effluent stream 212 with reduced CO, and a waste stream 214 comprising CO. One aspect can include further contaminant removal units, or a combined contaminant removal unit incorporated with the carbon oxide removal zone 210. The reduced CO effluent stream 212 is passed to a hydroprocessing zone 220 for upgrading the acetylene. One type of hydroprocessing zone 220 is a hydrogenation unit to convert the acetylene to ethylene and to generate a stream 222 comprising ethylene. Another type of hydroprocessing zone 220 includes a hydrogenation unit and a dimerization unit, to generate a stream 222 comprising ethylene and butene. The ethylene from the hydrogenation unit can be split into two portions with one portion passed to the dimerization unit. The dimerization unit can generate a butene stream and be combined with the ethylene stream to form a combined stream 222. The combined stream 222 is passed to a second hydrocarbon conversion zone 230 for further processing. The second hydrocarbon conversion zone 230 can comprise a metathesis zone to convert the ethylene and butene to propylene to generate a process stream 232. The process stream 232 is passed to a product recovery zone 240 to generate a light olefins stream 242, or a propylene stream 242, a heavy hydrocarbon stream 244, and other process streams (not shown). Alternative hydrocarbon conversion zones can include oligomerization units for generating heavier olefins, and multiple dimerization units for generating heavier olefins.

### Examples:

Example 1. A suitable oligomerization catalyst of the present invention was carried out by the coprecipitation of freshly prepared sodium aluminate and commercially available sodium silicate solutions by the addition of nitric acid. A typical synthesis of the catalyst entails a first preparation of sodium aluminate solution. To 7.5 ml of distilled water, 4.5 g of Al(OH)₃ and 5.0 g of NaOH was added and placed in a flask equipped with a condenser. The mixture was allowed to react at reflux, with stirring, until a clear solution was obtained. Of distilled water, 250 ml was then added to the flask and the solution stirred and heated for a further minute. The second stage of the typical synthesis entails preparation of a silica-alumina hydrogel. To 199 ml of waterglass solution (Merck, 28% by mass SiO₂) and 1085 ml of distilled water, 228 ml of the hot sodium aluminate solution was added, followed by addition of 1.4 M nitric acid under vigorous stirring to obtain a gel with a pH of 9 within 1-2 min. The hydrogel was then aged at 25°C for three days and then washed with distilled water until a neutral pH was obtained in the wash water. The third stage of the typical synthesis is the preparation of the solid silica-alumina. The diluted hydrogel obtained above was filtered using a Buchner funnel, to remove as much of the water as possible, and the more concentrated product then dried at 110°C overnight followed by calcination at 550°C for 3 hours. The Na+-form of the solid silica-alumina product with a silicon-to-aluminum ratio of 25 was thus obtained.

Nickel may be added to the silica-alumina solid support by ion exchange. Ion-exchange of the Na⁺-form of the solid support was effected by reflux with an aqueous solution of nickel chloride for 5 h using three moles of nickel(II) for every two moles of aluminum in the silica-alumina support. The green solids were then filtered and extensively washed with distilled water until the filtrates were free of chloride ions, otherwise detectable by the addition of silver nitrate. After drying at 110°C and following acid digestion of the green solids, the catalyst contained 1.56% nickel by mass as determined by atomic absorption spectroscopy, an aluminum content of 1.6 mass-%, a sodium content of 0.68 mass-%, a BET surface area of 425 m²/g, an average pore radius of 18.7Å, a pore volume of 0.75 cm³/g, and XRD analysis indicating an amorphous morphology. The catalyst may be activated by oxidation, but is not always necessary.

Example 2. We introduce a natural gas liquids stream comprising 87% CH4 to a supersonic methane pyrolysis reactor at a temperature of 2700°C and accelerate it to Mach 2.0 to give a 49% yield of acetylene comprising an acetylene stream. We pass the acetylene stream into a contaminant removal zone and remove carbon monoxide to a level of 30ppm. We pass the thus decontaminated acetylene stream to a hydrogenation zone. The catalyst in the hydrogenation zone comprises 0.3wt% Pt on alumina. The hydrogenation zone converts acetylene to ethylene with 99% efficiency to give an ethylene stream. The ethylene stream passes into an oligomerization zone. We use the catalyst of Example 1 to convert the ethylene stream to a product stream comprising primarily 1-hexene and 1-octene.

### SPECIFIC EMBODIMENTS

A first embodiment of the invention is a method for producing olefins comprising combusting a fuel and oxygen to produce a hot fluid carrier; introducing a hydrocarbon feed stream comprising methane into a supersonic reactor; mixing the carrier fluid and the hydrogen feedstream in a mixing zone; pyrolyzing the methane in the supersonic reactor to form a reactor effluent stream comprising acetylene; passing the reactor effluent stream to a first hydrocarbon conversion zone to form a second process stream comprising a second hydrocarbon compound; and passing the second process stream to a second hydrocarbon conversion zone to form a third process stream comprising C6 to C12 olefins. An embodiment of the invention is one, any or all of prior embodiments in this paragraph up through the first embodiment in this paragraph wherein the first hydrocarbon conversion zone comprises a hydroprocessing zone, and the second hydrocarbon conversion zone comprises an olefin conversion zone. An embodiment of the invention is one, any or all of prior embodiments in this paragraph up through the first embodiment in this paragraph wherein the olefin conversion zone is a oligomerization zone. An embodiment of the invention is one, any or all of prior embodiments in this paragraph up through the first embodiment in this paragraph wherein the oligomerization zone includes a catalyst comprising a Group VIIIB metal deposited on a silica-alumina support, and wherein the silica-alumina support has a silicon to aluminum ratio of at least 20. An embodiment of the invention is one, any or all of prior embodiments in this paragraph up through the first embodiment in this paragraph wherein the metal is nickel and the catalyst has a metal content between 0.5% and 10% by weight of the catalyst. An embodiment of the invention is one, any or all of prior embodiments in this paragraph up through the first embodiment in this paragraph wherein the oligomerization zone includes a catalyst comprising an organometallic catalyst. An embodiment of the invention is one, any or all of prior embodiments in this paragraph up through the first embodiment in this paragraph wherein the organometallic catalyst comprises Cr-PNP systems such as bis(diphenylphosphino)ethylamine Cr (III) chloride, Cr-SNS systems such as bis(dithioether)amine Cr (III) chloride, Cr-PNN systems such as diphenylphosphinotrimethylethylenediamine Cr (III) chloride, (Me3P)Cr[µ-(tBu)NPPh2]3Cr, chromium triazacyclohexane complexes, Ti cyclopentadienyl based systems such as 1,1'-dimethylbenzylcyclopentadienyltitanium trichloride and mixtures thereof. An embodiment of the invention is one, any or all of prior embodiments in this paragraph up through the first embodiment in this paragraph wherein the oligomerization zone is operated at a temperature between 25°C to 200°C. An embodiment of the invention is one, any or all of prior embodiments in this paragraph up through the first embodiment in this paragraph wherein the oligomerization zone is operated to generate an oligomerization effluent stream comprising 1-hexene, 1-octene, or a mixture of 1-hexene and 1-octene. An embodiment of the invention is one, any or all of prior embodiments in this paragraph up through the first embodiment in this paragraph wherein the olefin conversion zone is operated at conditions to generate an effluent stream comprising highly branched alkenes and having an aromatics content of less than 1% by weight. An embodiment of the invention is one, any or all of prior embodiments in this paragraph up through the first embodiment in this paragraph wherein the oligomerization zone includes a catalyst comprising a zeolite and a binder, wherein the zeolite has a structure selected from the group consisting of MFI, MEL, ITH, IMF, TUN, FER, BEA, FAU, BPH, MEI, MSE, MWW, UZM-8, MOR, OFF, MTW, TON, MTT, AFO, ATO, and AEL, and mixtures thereof, and wherein the catalyst has been treated with a phosphorous containing reagent selected from the group consisting of phosphate compounds, phosphite compounds, phosphorus oxytrichloride, and mixtures thereof, thereby forming a treated catalyst having a micropore volume less than 50% of, and a crystallinity greater than 50% of the untreated catalyst. An embodiment of the invention is one, any or all of prior embodiments in this paragraph up through the first embodiment in this paragraph wherein the zeolite has an MTT or an MTW zeolyte type structure. An embodiment of the invention is one, any or all of prior embodiments in this paragraph up through the first embodiment in this paragraph wherein the binder is alumina and wherein at least a portion of the Al₂O₃ binder is converted to a crystalline aluminum phosphate during the treatment with the phosphorous containing reagent. An embodiment of the invention is one, any or all of prior embodiments in this paragraph up through the first embodiment in this paragraph wherein the oligomerization zone is operated under reaction conditions that include a temperature between 70 and 300°C and the pressure is greater than 2MPa.

A second described embodiment of the invention is a method for producing olefins comprising the steps of introducing a hydrocarbon feed stream comprising methane into a supersonic reactor; pyrolyzing the methane in the supersonic reactor to form a reactor effluent stream comprising acetylene; passing the reactor effluent stream to a contaminant removal zone to reduce the carbon monoxide content and generate a treated reactor effluent stream; hydrogenating the treated reactor effluent stream in a hydroprocessing zone to form an effluent stream comprising ethylene; converting the effluent stream in an olefin conversion zone to a second process stream comprising C6-C12 olefins; and separating the second process stream from unconverted methane. A described embodiment is one, any or all of prior embodiments in this paragraph up through the second described embodiment in this paragraph, wherein pyrolyzing the methane includes accelerating the hydrocarbon stream to a velocity of between mach 1.0 and mach 4.0 and slowing down the hydrocarbon stream to increase the temperature of the hydrocarbon process stream A described embodiment is one, any or all of prior embodiments in this paragraph up through the second described embodiment in this paragraph, wherein pyrolyzing the methane includes heating the methane to a temperature of between 1200°C and 3500°C for a residence time of between 0.5 ms and 100 ms. A described embodiment is one, any or all of prior embodiments in this paragraph up through the second embodiment in this paragraph wherein the treated reactor effluent stream comprises less than 100 ppm carbon monoxide by volume. A described embodiment is one, any or all of prior embodiments in this paragraph up through the second described embodiment in this paragraph wherein the olefin conversion zone is an oligomerization zone and includes an oligomerization catalyst that is selected from the group consisting of a) an amorphous silica-alumina base with a metal from Group VIIIB and a silicon-to-aluminum ratio of no less than 20; b) an organometallic catalyst selected from the group consisting of a Cr-PNP system such as bis(diphenylphosphino)ethylamine Cr (III) chloride, a Cr-SNS system such as bis(dithioether)amine Cr (III) chloride, a Cr-PNN system such as diphenylphosphinotrimethylethylenediamine Cr (III) chloride, (Me3P)Cr[µ-(tBu)NPPh2]3Cr, a chromium triazacyclohexane complex, a Ti cyclopentadienyl based system such as 1,1'-dimethylbenzylcyclopentadienyltitanium trichloride and mixtures thereof; and c) a catalyst comprising a zeolite and an alumina binder, wherein the zeolite has a structure selected from the group consisting of MFI, MEL, ITH, IMF, TUN, FER, BEA, FAU, BPH, MEI, MSE, MWW, UZM-8, MOR, OFF, MTW, TON, MTT, AFO, ATO, and AEL, and mixtures thereof, wherein the catalyst has been treated with a phosphorous containing reagent selected from the group consisting of phosphate compounds, phosphite compounds, phosphorus oxytrichloride, and mixtures thereof, thereby forming a treated catalyst having a micropore volume less than 50% of, and a crystallinity greater than 50% of the untreated catalyst wherein at least a portion of the alumina binder has been converted to a crystalline aluminum phosphate during the phosphorous containing reagent treatment step. A described embodiment is one, any or all of prior embodiments in this paragraph up through the second described embodiment in this paragraph wherein the olefin conversion zone is an oligomerization zone operated under reaction conditions that include a temperature between 25°C and 300°C and the pressure is greater than 2MPa.

## Claims

1. A method for producing olefins comprising: combusting a fuel and oxygen to produce a hot carrier fluid; introducing a hydrocarbon feed stream comprising methane into a supersonic reactor; mixing the carrier fluid and the hydrocarbon feedstream in a mixing zone; pyrolyzing the methane in the supersonic reactor to form a reactor effluent stream comprising acetylene; passing the reactor effluent stream to a first hydrocarbon conversion zone to form a second process stream comprising a second hydrocarbon compound; and passing the second process stream to a second hydrocarbon conversion zone to form a third process stream comprising C6 to C12 olefins.

2. The method of claim 1 wherein the first hydrocarbon conversion zone comprises a hydroprocessing zone, and the second hydrocarbon conversion zone comprises an olefin conversion zone.

3. The method of claim 2 wherein the olefin conversion zone is a oligomerization zone.

4. The method of claim 3 wherein the oligomerization zone includes a catalyst comprising a Group VIIIB metal deposited on a silica-alumina support, and wherein the silica-alumina support has a silicon to aluminum ratio of at least 20.

5. The method of claim 4 wherein the metal is nickel and the catalyst has a metal content between 0.5% and 10% by weight of the catalyst.

6. The method of claim 3 wherein the oligomerization zone includes a catalyst comprising an organometallic catalyst, wherein the organometallic catalyst comprises Cr-PNP systems such as bis(diphenylphosphino)ethylamine Cr (III) chloride, Cr-SNS systems such as bis(dithioether)amine Cr (III) chloride, Cr-PNN systems such as diphenylphosphinotrimethylethylenediamine Cr (III) chloride, (Me3P)Cr[µ-(tBu)NPPh2]3Cr, chromium triazacyclohexane complexes, Ti cyclopentadienyl based systems such as 1,1'-dimethylbenzylcyclopentadienyltitanium trichloride and mixtures thereof.

7. The method of claim 3 wherein the oligomerization zone is operated at a temperature between 25°C to 300°C.

8. The method of claim 3 wherein the oligomerization zone is operated to generate an oligomerization effluent stream comprising 1-hexene, 1-octene, or a mixture of 1-hexene and 1-octene.

9. The method of claim 2 wherein the olefin conversion zone is operated at conditions to generate an effluent stream comprising highly branched alkenes and having an aromatics content of less than 1% by weight.

10. The method of claim 3 wherein the oligomerization zone includes a catalyst comprising a zeolite and a binder, wherein the zeolite has a structure selected from the group consisting of MFI, MEL, ITH, IMF, TUN, FER, BEA, FAU, BPH, MEI, MSE, MWW, UZM-8, MOR, OFF, MTW, TON, MTT, AFO, ATO, and AEL, and mixtures thereof, and wherein the catalyst has been treated with a phosphorous containing reagent selected from the group consisting of phosphate compounds, phosphite compounds, phosphorus oxytrichloride, and mixtures thereof, thereby forming a treated catalyst having a micropore volume less than 50% of, and a crystallinity greater than 50% of the untreated catalyst.

## Patentansprüche

1. Verfahren zur Herstellung von Olefinen, das Folgendes umfasst: Verbrennen eines Brennstoffs und Sauerstoffs, um eine heiße Trägerflüssigkeit zu erzeugen; Einleiten eines Methan umfassenden Kohlenwasserstoffzustroms in einen Überschallreaktor; Mischen der Trägerflüssigkeit und des Kohlenwasserstoffzustroms in einer Mischzone; Pyrolisieren des Methans im Überschallreaktor, um einen Reaktorabflussstrom zu bilden, der Acetylen umfasst; Leiten des Reaktorabflussstroms zu einer ersten Kohlenwasserstoffumwandlungszone, um einen zweiten Verfahrensstrom zu bilden, der eine zweite Kohlenwasserstoffverbindung umfasst; und Leiten des zweiten Verfahrensstroms zu einer zweiten Kohlenwasserstoffumwandlungszone, um einen dritten Verfahrensstrom zu bilden, der C6- bis C12-Olefine umfasst.

2. Verfahren nach Anspruch 1, wobei die erste Kohlenwasserstoffumwandlungszone eine Hydroverarbeitungszone umfasst, und die zweite Kohlenwasserstoffumwandlungszone eine Olefinumwandlungszone umfasst.

3. Verfahren nach Anspruch 2, wobei die Olefinumwandlungszone eine Oligomerisierungszone ist.

4. Verfahren nach Anspruch 3, wobei die Oligomerisierungszone einen Katalysator enthält, der eine Gruppe VIIIB Metall umfasst, das auf einem Siliziumdioxid-Aluminiumoxidträger abgelagert ist, und wobei der Siliziumdioxid-Aluminiumoxidträger ein Verhältnis von Silizium zu Aluminium von mindestens 20 aufweist.

5. Verfahren nach Anspruch 4, wobei das Metall Nickel ist und der Katalysator einen Metallgehalt zwischen 0,5 Gew.-% und 10 Gew.-% des Katalysators aufweist.

6. Verfahren nach Anspruch 3, wobei die Oligomerisierungszone einen Katalysator enthält, der einen metallorganischen Katalysator umfasst, wobei der metallorganische Katalysator Cr-PNP-Systeme wie bis(Diphenylphosphino)ethylamin-Cr-(III)-Chlorid, Cr-SNS Systeme wie bis(Dithioether)amin-Cr-(III)-Chlorid, Cr-PNN Systeme wie Diphenylphosphinotrimethylethylendiamin-Cr-(III)-Chlorid, (Me3P)Cr[p-(tBu)NPPh2]3Cr, Chrom-triazacyclohexan-Komplexe, Ti-Cyclopentadienyl-basierte Systeme wie 1,1'-Dimethylbenzylcyclopentadienyltitan-trichlorid und Mischungen davon umfasst.

7. Verfahren nach Anspruch 3, wobei die Oligomerisierungszone bei einer Temperatur zwischen 25 °C und 300 °C betrieben wird.

8. Verfahren nach Anspruch 3, wobei die Oligomerisierungszone betrieben wird, um einen Oligomerisierungsabflussstrom zu erzeugen umfassend 1-Hexen, 1 Octen oder eine Mischung von 1-Hexen und 1 Octen.

9. Verfahren nach Anspruch 2, wobei die Olefinumwandlungszone unter Bedingungen betrieben wird, die einen Abflussstrom erzeugen, der stark verzweigte Alkene umfasst und einen Aromatgehalt von weniger als 1 Gew.-% aufweist.

10. Verfahren nach Anspruch 3, wobei die Oligomerisierungszone einen Katalysator enthält, der ein Zeolith und ein Bindemittel umfasst, wobei das Zeolith eine Struktur aufweist, die aus der Gruppe ausgewählt ist bestehend aus MFI, MEL, ITH, IMF, TUN, FER, BEA, FAU, BPH, MEI, MSE, MWW, UZM-8, MOR, OFF, MTW, TON, MTT, AFO, ATO und AEL und Mischungen davon, und wobei der Katalysator mit einem phosphorhaltigen Reagens behandelt worden ist, das aus der Gruppe ausgewählt ist bestehend aus Phosphatverbindungen, Phosphitverbindungen, Phosphoroxytrichlorid und Mischungen davon, wodurch ein behandelter Katalysator gebildet wird, der ein Mikroporenvolumen von weniger als 50 % und eine Kristallinität von mehr als 50 % des unbehandelten Katalysators aufweist.

## Revendications

1. Procédé pour la production d'oléfines comprenant : la combustion d'un combustible et d'oxygène pour produire un fluide vecteur chaud ; l'introduction d'un flux d'alimentation d'hydrocarbures comprenant du méthane dans un réacteur supersonique ; le mélange du fluide vecteur et du flux d'alimentation d'hydrocarbures dans une zone de mélange ; la pyrolyse du méthane dans le réacteur supersonique pour former un flux d'effluent de réacteur comprenant de l'acétylène ; l'envoi du flux d'effluent de réacteur vers une première zone de conversion d'hydrocarbures pour former un deuxième flux de procédé comprenant un second composat d'hydrocarbures ; et l'envoi du deuxième flux de procédé vers une seconde zone de conversion d'hydrocarbures pour former un troisième flux de procédé comprenant des oléfines en C6 à C12.

2. Procédé selon la revendication 1 dans lequel la première zone de conversion d'hydrocarbures comprend une zone d'hydrotraitement et la seconde zone de conversion d'hydrocarbures comprend une zone de conversion d'oléfines.

3. Procédé selon la revendication 2 dans lequel la zone de conversion d'oléfines est une zone d'oligomérisation.

4. Procédé selon la revendication 3 dans lequel la zone d'oligomérisation comprend un catalyseur comprenant un métal du groupe VIIIB déposé sur un support en silice-alumine et dans lequel le support en silice-alumine a un rapport du silicium à l'aluminium d'au moins 20.

5. Procédé selon la revendication 4 dans lequel le métal est le nickel et le catalyseur a une teneur en métal entre 0,5 % et 10 % en poids du catalyseur.

6. Procédé selon la revendication 3 dans lequel la zone d'oligomérisation comprend un catalyseur comprenant un catalyseur organométallique, le catalyseur organométallique comprenant des systèmes Cr-PNP tels que le chlorure de bis(diphénylphosphino)éthylamine-Cr(III), des systèmes Cr-SNS tels que le chlorure de bis(dithioéther)amine-Cr(III), des systèmes Cr-PNN tels que le chlorure de diphénylphosphinotriméthyléthylènediamine-Cr(III), (Me₃P)Cr[µ-(tBu)NPPh₂]₃Cr, des complexes de triazacyclohexane et de chrome, des systèmes à base de cyclopentadiényle et de Ti tels que le trichlorure de 1,1'-diméthylbenzylcyclopentadiényltitane et des mélanges de ceux-ci.

7. Procédé selon la revendication 3 dans lequel la zone d'oligomérisation est amenée à fonctionner à une température entre 25 °C et 300 °C.

8. Procédé selon la revendication 3 dans lequel la zone d'oligomérisation est amenée à fonctionner pour produire un flux d'effluent d'oligomérisation comprenant de l'hex-1-ène, de l'oct-1-ène ou un mélange d'hex-1-ène et d'oct-1-ène.

9. Procédé selon la revendication 2 dans lequel la zone de conversion d'oléfines est amenée à fonctionner dans des conditions pour produire un flux d'effluent comprenant des alcènes hautement ramifiés et ayant une teneur en composés aromatiques inférieure à 1 % en poids.

10. Procédé selon la revendication 3 dans lequel la zone d'oligomérisation comprend un catalyseur comprenant une zéolite et un liant, la zéolite ayant une structure choisie dans le groupe constitué par les structures MFI, MEL, ITH, IMF, TUN, FER, BEA, FAU, BPH, MEI, MSE, MWW, UZM-8, MOR, OFF, MTW, TON, MTT, AFO, ATO et AEL et les mélanges de celles-ci et le catalyseur ayant été traité avec un réactif contenant du phosphore choisi dans le groupe constitué par les composés phosphates, les composés phosphites, l'oxytrichlorure de phosphore et les mélanges de ceux-ci, formant ainsi un catalyseur traité ayant un volume des micropores inférieur à 50 % du catalyseur non traité et une cristallinité supérieure à 50 % du catalyseur non traité.
